# EUROPÄISCHE PATENTSCHRIFT

(11) **EP 3 266 394 B1**
(45) Veröffentlichungstag und Bekanntmachung des Hinweises auf die Patenterteilung: **05.12.2018**
(21) Anmeldenummer: 16178549.8
(22) Anmeldetag: 08.07.2016
(51) Int. Cl.: A61B 17/29, A61B 17/30

(54) **CHIRURGISCHES INSTRUMENT MIT EINER VERRIEGELUNGSEINRICHTUNG**
SURGICAL INSTRUMENT WITH A LOCKING DEVICE
INSTRUMENT CHIRURGICAL COMPRENANT UN DISPOSITIF DE VERROUILLAGE

(43) Veröffentlichungstag der Anmeldung: 10.01.2018
(73) Patentinhaber: Tschida, Peter, 78580 Bärenthal (DE)
(72) Erfinder: Tschida, Peter, 78580 Bärenthal (DE)
(74) Vertreter: Späth, Dieter

(56) Entgegenhaltungen:
- EP-B1- 1 897 505
- DE-A1-102011 081 344
- US-A- 5 409 478
- US-A- 5 792 178
- US-A1- 2006 190 035
- US-B1- 6 322 578

## Beschreibung

Die Erfindung betrifft ein chirurgisches Instrument mit einer lösbaren mechanischen Verriegelungseinrichtung mit den Merkmalen des Anspruchs 1. Vorteilhafte Ausgestaltungen sind Gegenstand der Unteransprüche. Die Verriegelungseinrichtung ist insbesondere dazu vorgesehen, das chirurgische Instrument in einer betätigten Stellung zu verriegeln. Das chirurgische Instrument ist zu einer Verwendung in der minimal-invasiven Chirurgie vorgesehen.

Ein derartiges chirurgisches Instrument ist bekannt aus dem europäischen Patent EP 1 897 505 B1. Das bekannte chirurgische Instrument weist einen Hohlschaft, in dem eine Seele verschiebbar ist, und eine Bedieneinheit zu einer Betätigung des Instruments von Hand auf. Durch eine lösbare Kupplung ist die Bedieneinheit mit der Seele verbunden, um den Hohlschaft durch Schrauben und die Seele durch Lösen der Kupplung zum Spülen von der Bedieneinheit lösen zu können.

Die Bedieneinheit des bekannten chirurgischen Instruments weist zwei Griffe auf, die V-förmig schräg in einem spitzen Winkel zueinander stehen und zur Betätigung des chirurgischen Instruments zusammendrückbar sind. Über zwei Schwenkhebel, deren äußere Enden gelenkig mit einander fernen Enden der Griffe und deren innere Enden gemeinsam gelenkig mit der Kupplung verbunden sind, wird beim Zusammendrücken der Griffe die Seele im Hohlschaft verschoben.

Eine Verriegelungseinrichtung des bekannten chirurgischen Instruments weist einen Schaft auf, der senkrecht von einem der beiden Griffe in Richtung des anderen Griffs absteht. Ein Fuß des Schafts ist in einer Längsmitte einer Blattfeder befestigt, die in einer Ausnehmung des einen Griffs parallel zu dem einen Griff angeordnet ist und deren Enden an dem einen Griff befestigt sind. An einem freien, dem einen Griff fernen Ende weist der Schaft der Verriegelungseinrichtung des bekannten chirurgischen Instruments einen Riegel in der Form eines spitzwinkligen und zu einer Seite gekrümmten Dreiecks auf, der mit einem Stift zusammenwirkt, der quer in einer Ausnehmung des anderen Griffs angeordnet ist. Beim Zusammendrücken der beiden Griffe stößt eine schräg zu den Griffen verlaufende Grundseite des dreieckigen Riegels am freien Ende des Schafts der Verriegelungseinrichtung gegen den Stift in der Ausnehmung des anderen Griffs und drückt den Schaft zur Seite, so dass der Riegel am Stift vorbei treten kann. Die Blattfeder, in deren Längsmitte der Fuß des Schafts befestigt ist, wird dabei elastisch verformt. Der am Stift vorbeitretende Riegel hintergreift den Stift mit einer konkaven Seite, wodurch die beiden Griffe zusammengehalten und das chirurgische Instrument in betätigter Stellung gehalten wird, was auch als Verriegelung in betätigter Stellung aufgefasst werden kann.

Zum Lösen werden die beiden Griffe weiter zusammengedrückt, bis der Stift in der Ausnehmung des anderen Griffs an einer dem einen Griff zugewandten Spitze des dreieckigen Riegels vorbei tritt und bei einem anschließenden Loslassen der beiden Griffe an einer konvexen Seite des dreieckigen Riegels entlang gleitet und vom Riegel freikommt. Das weitere Zusammendrücken der beiden verriegelten Griffe zum Lösen der Verriegelung kann auch als Überdrücken der Griffe aufgefasst werden.

Die Patentanmeldung US 2006/0 190 035 A1 offenbart ein chirurgisches Instrument mit zwei ebenfalls V-förmig in einem spitzen Winkel zueinander angeordneten Griffen, die zusammen und auseinander schwenkbar sind. Die Griffe sind symmetrisch zu einem Hohlschaft des Instruments angeordnet, in dem eine Seele verschiebbar ist. Über je einen Hebel, deren eine Enden gelenkig mit den Griffen und deren andere Enden gelenkig mit der Seele verbunden sind, lässt sich durch Schwenken der Griffe die Seele in dem Hohlschaft verschieben. Eine Rückstellfeder, die sich axial an dem Hohlschaft abstützt und gegen die Seele drückt, schwenkt die Griffe über die Hebel auseinander und bewegt das chirurgische Instrument in eine Ausgangsstellung. Die Griffe lassen sich so weit zusammen drücken, dass die Hebel einen Totpunkt überwinden, so dass die Rückstellfeder die Griffe nach innen auf eine Achse des Hohlschafts und der Seele zu anstatt nach außen schwenkt. Dadurch hält die Rückstellfeder das chirurgische Instrument in einer betätigten Stellung, wenn die Griffe über ihren Totpunkt nach innen geschwenkt worden sind. Zum Öffnen des bekannten chirurgischen Instruments müssen die Griffe so weit nach außen geschwenkt werden, dass sie ihren Totpunkt von innen nach außen überwinden, woraufhin die Rückstellfeder die Griffe über die Hebel wieder nach außen beaufschlagt.

Die Patentanmeldung US 5 792 178 A offenbart ein chirurgisches Instrument mit einem V-förmig in einem spitzen Winkel von einem Grundkörper abstehenden Griff, dessen eines Ende schwenkbar an dem Grundkörper gelagert ist. Ein entgegengesetzt schräg vom Griff in den Grundkörper abstehender Hebel, dessen eines Ende gelenkig mit dem Griff verbunden ist, stützt sich schwenkbar an einer Seele ab, die verschiebbar in dem Grundkörper angeordnet ist. Wird der Griff zum Grundkörper geschwenkt, verschiebt er über den Hebel die Seele in ihrer Längsrichtung. Mit gleicher Schwenkachse wie der Griff ist ein weiterer Hebel schwenkbar an dem Grundkörper gelagert, der einen Riegel bildet und der federbeaufschlagt in eine rechteckige Aussparung des Hebels einrastet, wenn der Griff und über den Griff der Hebel nach innen, das heißt in Richtung des Grundkörpers gedrückt werden. Der in die Aussparung des Hebels eingerastete Riegel bildet eine mechanische Verriegelungseinrichtung, die den Griff und den Hebel in der nach innen zum Grundkörper geschwenkten und die Seele in einer verschobenen Stellung hält. Zum Ausrasten muss der Riegel aus der Aussparung des Hebels heraus bewegt werden, wofür eine Taste vorgesehen ist.

Aufgabe der Erfindung ist ein chirurgisches Instrument mit einer Bedieneinheit zu einer Betätigung von Hand und mit einer mechanischen Verriegelungseinrichtung vorzuschlagen, die zuverlässig verriegelt.

Gelöst wird diese Aufgabe durch ein chirurgisches Instrument mit den Merkmalen des Anspruchs 1. Das erfindungsgemäße chirurgische Instrument ist zur Verwendung in der minimal-invasiven Chirurgie vorgesehen. Es weist eine Bedieneinheit zu einer Betätigung des Instruments von Hand auf. Die Bedieneinheit weist einen Grundkörper, der auch Grundkörper oder Teil eines Grundkörpers des chirurgischen Instruments insgesamt sein kann, einen in Bezug auf den Grundkörper beweglichen Griff, ein in Bezug auf den Grundkörper bewegliches Übertragungselement und ein Übertragungsglied, das zur Übertragung einer Bewegung des Griffs auf das Übertragungselement mit dem Griff und mit dem Übertragungselement gekoppelt ist, auf. Durch die Beweglichkeit in Bezug zum Grundkörper sind der Griff, das Übertragungselement und das Übertragungsglied auch in Bezug zueinander beweglich.

Bewegungsarten des Griffs, des Übertragungselements und des Übertragungsglieds, nämlich translatorisch und/oder rotatorisch, und Bewegungsrichtungen können verschieden sein und sind vorzugsweise verschieden. Das Übertragungselement ist vorzugsweise in einer Längsrichtung des Grundkörpers oder eines Schafts des chirurgischen Instruments verschiebbar am oder im Grundkörper bzw. Schaft geführt. Es überträgt seine Bewegung auf ein Arbeitselement des chirurgischen Instruments. Das Arbeitselement ist beispielsweise eine Klinge einer Schere, ein Schenkel einer Pinzette oder eine Backe eines Nadelhalters oder einer Zange, das das Übertragungselement bei seiner Bewegung verschwenkt. Der Griff ist vorzugsweise schwenkbar mit dem Grundkörper verbunden und/oder das Übertragungsglied gelenkig mit dem Griff und/oder gelenkig mit dem Übertragungselement verbunden.

Erfindungsgemäß ist ein Verriegelungselement, beispielsweise ein Riegel der Verriegelungseinrichtung, an einem Schwenkhebel angeordnet, der schwenkbar an dem Übertragungsglied gelagert ist. Ein Gegenstück des Verriegelungselements kann ebenfalls an einem Schwenkhebel angeordnet sein, der schwenkbar an dem anderen Übertragungsglied gelagert ist. Bei einem Querversatz des Griffs durch seitliches Spiel und/oder Verformung ist eine Abweichung des Verriegelungselements zu einer Seite kleiner, wenn das Verriegelungselement erfindungsgemäß am Übertragungsglied und nicht am Griff angeordnet ist. Das gilt jedenfalls dann, wenn sich das Übertragungsglied näher am
Grundkörper befindet als der Griff, was typischerweise bei vielen chirurgischen Instrumenten mit einer gattungsgemäßen Bedieneinheit der Fall ist. Der Schwenkhebel ermöglicht eine Bewegung des Verriegelungselements, um das Verriegelungselement zum Verriegeln in Eingriff oder in Hintergriff am Gegenstück und um das Verriegelungselement zum Lösen der Verriegelung außer Eingriff vom Gegenstück zu bringen.

Die Bedieneinheit kann gerade, abgewinkelt oder gekröpft, also mit Versatz zu einer Seite, am chirurgischen Instrument angeordnet sein. Die Bedieneinheit kann vergleichbar einem Pistolengriff mit dem Grundkörper als feststehender Handgriff und dem Griff anstelle eines Abzugs ausgebildet sein.

Eine Ausgestaltung der Erfindung sieht zwei bewegliche Griffe vor, die entgegengesetzt zueinander und die in Bezug auf den Grundkörper beweglich sind. Insbesondere sind die beiden Griffe symmetrisch zueinander und zu dem zwischen ihnen angeordneten Grundkörper beweglich. Des Weiteren weist diese Ausgestaltung der Erfindung zwei Übertragungsglieder auf, von denen jedes mit einem Griff und die beide mit dem Übertragungselement gekoppelt sind und Bewegungen der Griffe auf das Übertragungselement übertragen. Die beiden Griffe können vergleichbar den Schenkeln einer Pinzette oder einer Zange angeordnet sein.

Eine Weiterbildung der Ausgestaltung der Erfindung mit zwei beweglichen Griffen sieht vor, dass das Verriegelungselement an einem der beiden Übertragungsglieder und ein Gegenstück des Verriegelungselements an einem anderen der beiden Übertragungsglieder angeordnet ist. Das Verriegelungselement ist beispielsweise ein Riegel, der bei einem Verriegeln in Eingriff mit dem Gegenstück gelangt, das ebenfalls ein Riegel oder ein anderes Bauteil sein kann, und der in verriegeltem Zustand mit dem Gegenstück in Eingriff steht und/oder das Gegenstück hintergreift. Insbesondere bei zwei beweglichen Griffen ist eine seitliche Bewegung durch Spiel und/oder Verformung größer als an den näher beieinander befindlichen Übertragungsgliedern, so dass die Verriegelungseinrichtung zuverlässiger verriegelt, wenn ihr Verriegelungselement und das Gegenstück an den beiden Übertragungsgliedern anstatt an den Griffen angeordnet sind.

Eine bevorzugte Ausgestaltung der Erfindung sieht ein Zwischenglied zwischen dem Griff und dem Übertragungsglied vor, das mit dem Griff und dem Übertragungsglied gekoppelt ist und eine Bewegung des Griffs über das Übertragungsglied auf das Übertragungselement überträgt, das bei seiner Bewegung das Arbeitselement, beispielsweise eine Klinge einer Schere, einen Schenkel einer Pinzette oder eine Backe einer Zange, des chirurgischen Instruments bewegt. Beispielsweise ist das Zwischenglied gelenkig mit dem Griff und/oder gelenkig mit dem Übertragungsglied verbunden. Ein Vorteil dieser Ausgestaltung der Erfindung ist, dass das Übertragungsglied durch das Zwischenglied stärker von einer seitlichen Bewegung des Griffs entkoppelt ist. Wird der Griff seitlich, das heißt quer zu seiner vorgesehenen Bewegungsrichtung beaufschlagt, wird das Übertragungsglied aufgrund des Zwischenglieds weniger stark zur Seite gedrückt als ohne Zwischenglied. Das erhöht die Zuverlässigkeit des Verriegelns, weil das Verriegelungselement am Übertragungsglied angeordnet ist. Es wird wegen des Zwischenglieds bei einer seitlichen Beaufschlagung des Griffs weniger weit aus einer vorgesehenen Bewegungsbahn gedrückt.

Eine weitere bevorzugte Ausgestaltung der Erfindung sieht einen Schlitz im Grundkörper vor, in dem das oder die Übertragungsglieder zumindest teilweise aufgenommen sind. Der Schlitz im Grundkörper ist so breit wie das Übertragungsglied dick ist oder die beiden Übertragungsglieder zusammen dick sind. Der Schlitz im Grundkörper stützt die Übertragungsglieder an einander abgewandten Seiten ab und hält sie in Anlage an einander, wodurch sicher gestellt ist, dass das an einem Übertragungsglied angeordnete Verriegelungselement beim Verriegeln
in Eingriff oder in Hintergriff an dem Gegenstück gelangt und nicht durch ein Auseinanderbiegen der beiden Übertragungsglieder seitlich am Gegenstück vorbei tritt.

Die Erfindung wird nachfolgend anhand zweier in der Zeichnung dargestellter Ausführungsbeispiele näher erläutert. Es zeigen:
- Figur 1: eine Seitenansicht eines chirurgischen Instruments gemäß der Erfindung;
- Figur 2: ein Hebelgetriebe des chirurgischen Instruments aus Figur 1 in Seitenansicht;
- Figur 3: Teile des Hebelgetriebes aus Figur 2 in vergrößerter, perspektivischer Darstellung;
- Figur 4: eine in das Hebelgetriebe aus Figur 3 integrierte Verriegelungseinrichtung in perspektivischer Darstellung,
- Figur 5: ein zweites Ausführungsbeispiel eines erfindungsgemäßen chirurgischen Instruments in perspektivischer Darstellung; und
- Figur 6: eine vergrößerte Einzelheit gemäß Kreis VI in Figur 5.

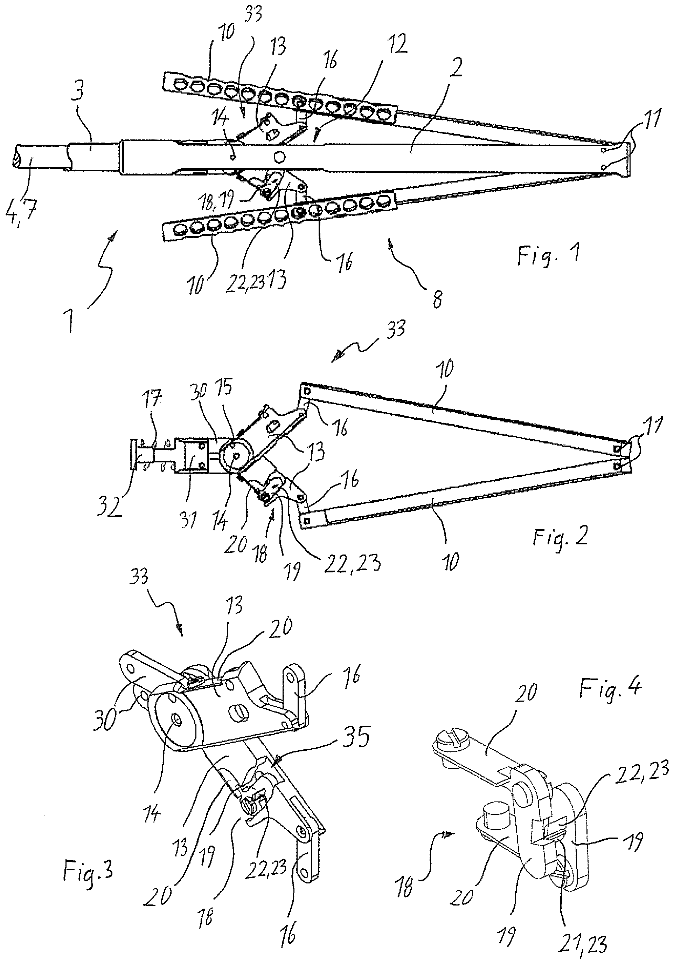

Das in Figur 1 dargestellte, erfindungsgemäße chirurgische Instrument 1 ist zur Verwendung in der minimal-invasiven Chirurgie vorgesehen. Es weist ein Rohr als Grundkörper 2 und ein weiteres Rohr mit kleinerem Durchmesser als Hohlschaft 3 auf, der gleichachsig und starr mit dem Grundkörper 2 verbunden an dessen einem Ende angeordnet ist. In dem Hohlschaft 3 ist eine Stange als Seele 4 verschiebbar aufgenommen. Im dargestellten und beschriebenen Ausführungsbeispiel der Erfindung ist das chirurgische Instrument 1 eine Schere, es weist an einem dem Grundkörper 2 fernen Ende des Hohlschafts 3 eine feststehende Klinge und eine schwenkbare Klinge auf, die schwenkbar am Hohlschaft 3 gelagert und exzentrisch zu ihrer Schwenklagerung gelenkig mit der Seele 4 verbunden ist, so dass die schwenkbare Klinge durch Verschieben der Seele 4 im Hohlschaft 3 schwenkbar ist. Das dem Grundkörper 2 ferne Ende des chirurgischen Instrumenst 1 ist nicht gezeichnet. Es sind auch andere Ausführungen des chirurgischen Instruments 1 beispielsweise als Pinzette, Nadelhalter oder Zange und mit zwei schwenkbaren Klingen, Schenkeln oder Backen anstatt einer feststehenden und einer schwenkbaren Klinge, Schenkel oder Backe möglich (nicht dargestellt). Die Seele 4 kann allgemein auch als Übertragungselement 7, das eine Bewegung von einer noch zu erläuternden Bedieneinheit 8 auf die schwenkbare Klinge überträgt, und die schwenkbare Klinge als Arbeitselement des chirurgischen Instruments 1 aufgefasst werden.

Die Bedieneinheit 8 ist an einem den Klingen fernen Ende des Hohlschafts 3 angeordnet, sie dient einer Betätigung des Instruments 1 von Hand. Der Grundkörper 2 ist Bestandteil des chirurgischen Instruments 1 und der Bedieneinheit 8. Die Bedieneinheit 8 weist zwei Griffe 10 auf, die V-förmig in einem spitzen Winkel schräg von dem den Grundkörper 2 bildenden Rohr abstehen. Der Grundkörper 2 befindet sich in einer Mitte zwischen den beiden Griffen 10, die synchron einander entgegengesetzt zum Grundkörper 2 und zurück in ihre schräg vom Grundkörper 2 abstehende Stellung bewegbar sind. Im Ausführungsbeispiel sind die Griffe 10 halbzylinderschalenförmig und es sind Enden der Griffe 10 mit Stiften 11 schwenkbar mit dem Grundkörper 2 verbunden. Im Ausführungsbeispiel der Erfindung stehen die beiden Griffe 10 in Richtung des Hohlschafts 3 schräg nach außen vom Grundkörper 2 ab und es sind dem Hohlschaft 3 ferne Enden der Griffe 10 mit den Stiften 11 schwenkbar mit einem dem Hohlschaft 3 fernen Ende des Grundkörpers 2 verbunden. Die Griffe 10 können allerdings ebenso gut umgekehrt in Richtung weg vom Hohlschaft 3 schräg nach außen vom Grundkörper 2 abstehen und ihre dem Hohlschaft 3 nahen Enden schwenkbar mit dem Grundkörper 2 verbunden sein (nicht dargestellt). Ebenfalls abweichend von der Zeichnung kann die Bedieneinheit 8 in einem Winkel und/oder versetzt anstatt gleichachsig zum Hohlschaft 3 angeordnet sein.

Der Grundkörper 2 weist einen Schlitz 12 in einer Axialebene auf, in dem zwei Schwenkhebel als Übertragungsglieder 13 aufgenommen sind. Die die Übertragungsglieder 13 bildenden Schwenkhebel sind leistenförmig und liegen mit einander zugewandten Flachseiten aneinander an. Der Schlitz 12 im Grundkörper 2 ist so breit wie die beiden Übertragungsglieder 13 zusammen dick sind, so dass der Schlitz 12 die Übertragungsglieder 13 von außen abstützt und ihre einander zugewandten Flachseiten in Anlage aneinander hält. Die Übertragungsglieder 13 sind mit einem Stift 14 gemeinsam schwenkbar in einer zum Schlitz 12 senkrechten Axialebene im Grundkörper 2 gelagert und mit jeweils einem weiteren Stift 15 über Zwischenhebel 30, ein Joch 31, das eine Kupplung 32 zur Verbindung mit der Seele 4 aufweist, gelenkig mit der Seele 4 verbunden, die das Übertragungselement 7 zur Übertragung einer Bewegung von der Bedieneinheit 8 auf die schwenkbare Klinge bzw. das Arbeitselement des chirurgischen Instruments 1 bildet. Die Zwischenhebel 30 sind gelenkig mit den Übertragungsgliedern 13 und dem Joch 31 verbunden. Die Zwischenhebel 30, das Joch 31 und die Kupplung 32 sind in dem Schlitz 12 im Grundkörper 2 angeordnet. Die Griffe 10, Zwischenglieder 16, Übertragungsglieder 13, Zwischenhebel 30 und das Joch 31 bilden ein ebenes Hebelgetriebe 33, dass in Figur 2 dargestellt ist. Zusätzlich sind die Zwischenglieder 16, Übertragungsglieder 13 und Zwischenhebel 30 des Hebelgetriebes 33 vergrößert und perspektivisch in Figur 3 dargestellt.
Den Stiften 14, 15, die die Übertragungsglieder 13 schwenkbar mit dem Grundkörper 2 und mit der Seele 4 verbinden, ferne Enden der Übertragungsglieder 13 sind über Schwenkhebel als Zwischenglieder 16 gelenkig mit den Griffen 10 verbunden. Im Ausführungsbeispiel sind die Zwischenglieder 16 etwa in Längsmitten der Griffe 10 angelenkt und stehen rechtwinklig zum Grundkörper 2 nach innen. Es sind allerdings andere Geometrien möglich.
Bei unbetätigtem chirurgischen Instrument 1 stehen die beiden Griffe 10 wie bereits beschrieben V-förmig in einem spitzen Winkel schräg nach außen vom Grundkörper 2 ab. Ebenfalls stehen die beiden Übertragungsglieder 13 V-förmig schräg nach außen vom Grundkörper 2 ab, allerdings im Ausführungsbeispiel in entgegengesetzter Richtung wie die Griffe 10. Zur Betätigung des chirurgischen Instruments 1 werden die Griffe 10 nach innen, d. h. auf einander zu und zum Grundkörper 2, gedrückt. Über die Zwischenglieder 16 drücken die Griffe 10 die Enden der Übertragungsglieder 13, die über die Zwischenglieder 16 mit den Griffen 10 verbunden sind, nach innen in den Schlitz 12 des Grundkörpers 2. Dabei schwenken die Übertragungsglieder 13 um ihren gemeinsamen Stift 14, der sie gelenkig im Grundkörper 2 lagert, und verschieben über die Zwischenhebel 30, das Joch 31 und die Kupplung 32 die Seele 4 im Hohlschaft 3. Durch ihre Verschiebung im Hohlschaft 3 schwenkt die Seele 4, die das Übertragungselement 7 bildet, die schwenkbare Klinge, die das Arbeitselement des chirurgischen Instruments 1 bildet, an dem der Bedieneinheit 8 fernen Ende des Hohlschaft 3. Eine Rückstellfeder 17 beaufschlagt die Seele 4 und die Übertragungsglieder 13, die Zwischenglieder 16 und die Griffe 10 in entgegengesetzter Richtung und stellt sie nach Loslassen der Griffe 10 zurück in ihre Ausgangsstellungen. Im Ausführungsbeispiel ist die Rückstellfeder 17 eine Schraubendruckfeder, die auf der Kupplung 32 in dem den Grundkörper 2 bildenden Rohr angeordnet ist, sich im Grundkörper 2 abstützt und gegen das Joch 31 drückt.
Die Bedieneinheit 8 des erfindungsgemäßen chirurgischen Instruments 1 weist eine lösbare mechanische Verriegelungseinrichtung 18 auf, die an den beiden Übertragungsgliedern 13 angeordnet ist: die Verriegelungseinrichtung 18 weist an jedem der beiden Übertragungsglieder 13 einen Schwenkhebel 19 auf, die im Ausführungsbeispiel in Aussparungen 35 in den einander zugewandten und aneinander anliegenden Flachseiten der Übertragungsglieder 13 angeordnet sind (Figur 3). Die Schwenkhebel 19 werden von Blattfedern als Riegelfedern 20, die an den Übertragungsgliedern 13 befestigt sind, in eine noch zu erläuternde verriegelte Stellung beaufschlagt. An ihren einander zugewandten Seiten weist einer der Schwenkhebel 19 einen Rasthaken 21 auf, der mit einem Riegel 22 zusammenwirkt, der vom andern Schwenkhebel 19 der Verriegelungseinrichtung 18 absteht. Der Rasthaken 21 und der Riegel 22 können auch als Verriegelungselemente 23 oder als Verriegelungselement 23 und Gegenstück des Verriegelungselements 23 aufgefasst werden. Die Verriegelungseinrichtung 18 mit den Schwenkhebeln 19, Riegelfedern 20, Rasthaken 21, Riegel 22, Verriegelungselement 23 sind ohne die Übertragungsglieder 13 in Figur 4 in der verriegelten Stellung gezeichnet.

Werden die beiden Übertragungsglieder 13 durch Zusammendrücken der Griffe 10 nach innen in den Schlitz 12 im Grundkörper 2 geschwenkt, stoßen die Verriegelungselemente 23 gegeneinander. Der Rasthaken 21 ist so geformt und angeordnet, dass er gegen den Riegel 22 stößt, wenn die Übertragungsglieder 13 in den Schlitz 12 im Grundkörper 2 geschwenkt werden. Beim Zusammenstoßen lenken der Rasthaken 21 und der Riegel 22 die Schwenkhebel 19, von denen sie seitlich und zueinander abstehen, aus, so dass der Rasthaken 21 am Riegel 22 vorbei tritt. Werden die Griffe 10 anschließend losgelassen, gelangt der Riegel 22 in Eingriff bzw. Hintergriff am bzw. im Rasthaken 21, so dass der Rasthaken 21 und der Riegel 22, die die Verriegelungselemente 23 bzw. ein Verriegelungselement 23 und ein Gegenstück bilden, die Übertragungsglieder 13 innen in der betätigten Stellung und über die Seele 4 die schwenkbare Klinge in ihrer verschwenkten Stellung halten. Die Schere ist geschlossen. Der Eingriff bzw. Hintergriff des Riegels 22 am bzw. im Rasthaken 21 ist die verriegelte Stellung, die Figur 4 zeigt. Die Ausbildung des Rasthakens 21 und des Riegels 22 ist als sogenannte Push-Push-Verriegelungsmechanik oder aufgrund der Form des Rasthakens 21 als Herzkurvensteuerung beispielsweise von Kugelschreibern bekannt.

Zum Lösen der Verriegelung werden die Griffe 10 ein kurzes Stück zusammengedrückt, wodurch die beiden Verriegelungselemente 23 außer Eingriff voneinander gelangen. Die Riegelfedern 20, die beim Verriegeln elastisch verformt worden sind, schwenken die Schwenkhebel 19 so, dass bei einem anschließenden Loslassen der Griffe 10 die Verriegelungselemente 23 aneinander vorbei treten. Die Griffe 10 und die Übertragungsglieder 13 schwenken beaufschlagt von der Rückstellfeder 17 nach außen und die Seele 4 verschiebt sich im Hohlschaft 3 zurück in ihre Ausgangslage und schwenkt die schwenkbare Klinge in eine offene, schräg von der feststehenden Klinge abstehende Ausgangsstellung.

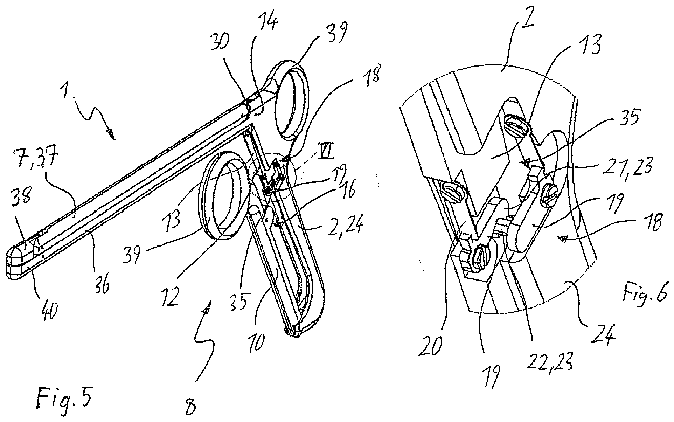

Figur 5 zeigt ein zweites Ausführungsbeispiel eines erfindungsgemäßen chirurgischen Instruments 1. Im Unterschied zu Figuren 1 und 2 ist eine Bedieneinheit 8 des chirurgischen Instruments 1 aus Figur 5 nicht symmetrisch aufgebaut sondern weist nur einen beweglichen Griff 10, ein Übertragungsglied 13 und ein Zwischenglied 16 auf. Ebenfalls abweichend von Figuren 1 und 2 weist das chirurgische Instrument 1 aus Figur 5 keinen Hohlschaft, sondern einen sogenannten Schiebeschaft auf. Der Schiebeschaft umfasst einen Schaft 36 und eine Schiebestange 37, die beispielsweise mit einer T-Nut verschiebbar am Schaft 36 geführt ist. Der Schaft 36 ist starr mit einem Grundkörper 2 des chirurgischen Instruments 1 bzw. einer Bedieneinheit 8 des chirurgischen Instruments 1, im Ausführungsbeispiel ist der Schaft 36 einstückig mit dem Grundkörper 2. Der Grundkörper 2 steht als Handgriff 24 wie ein Pistolengriff schräg in einem Winkel vom Schaft 36 ab. Der Winkel zwischen dem den Handgriff 24 bildenden Grundkörper 2 und dem Schaft 36 ist nicht maßgeblich für die Erfindung, der Handgriff 24 kann beispielsweise auch rechtwinklig oder in einem anderen Winkel vom Schaft 36 abstehen oder auch wie in Figuren 1 und 2 gleichachsig oder parallel versetzt zum Schaft 36 angeordnet sein (nicht dargestellt).

Der bewegliche Griff 10 ist an einem dem Schaft 36 fernen Ende des den Handgriff 24 bildenden Grundkörpers 2 schwenkbar gelagert, wobei abweichend auch eine Schwenklagerung nahe dem Schaft 36 am Grundteil 2 oder auch am Schaft 36 selbst oder auch eine andere Bewegungsmöglichkeit des Griffs 10 in Bezug zum Handgriff 24 möglich ist (nicht dargestellt). Ein Ende eines leistenförmigen Übertragungsglieds 13 ist in einem Übergangsbereich vom Handgriff 24 zum Schaft 36 mit einem Stift 14 schwenkbar gelagert. Der Handgriff 24 weist einen Schlitz 12 auf, in dem das schwenkbar gelagerte Ende des Übertragungsglied 13 aufgenommen ist und in den das Übertragungsglied 13 bei einer Betätigung des chirurgischen Instruments 1 versenkt wird. Der Schlitz 12 ist so breit wie das Übertragungsglied 13, so dass das Übertragungsglied 13 seitlich im Handgriff 24 geführt und abgestützt ist. Ein dem Schaft 36 fernes Ende des Übertragungsglieds 13 ist gelenkig mit einem Ende des Zwischenglieds 16 verbunden, dessen anderes Ende gelenkig mit dem Griff 10 verbunden ist. Über einen Zwischenhebel 30, der mit Abstand vom Stift 14 gelenkig mit dem Übertragungsglied 13 und dessen anderes Ende gelenkig mit der Schiebestange 37 verbunden ist, die das Übertragungselement 7 bildet, ist das Übertragungsglied 13 gelenkig mit der Schiebestange 37 verbunden. Wird der Griff 10 zum Handgriff 24 geschwenkt oder allgemein bewegt, verschwenkt er über das Zwischenglied 16 das Übertragungsglied 13, das über den Zwischenhebel 30 die Schiebestange 37 entlang des Schafts 36 verschiebt. Die Verschiebung der Schiebestange 37 gegenüber dem Schaft 36 schwenkt eine Klinge 38 einer Schere, die schwenkbar am Schaft 36 gelagert und mit Abstand von ihrer Schwenklagerung gelenkig mit der Schiebestange 37 verbunden ist. Zu einer Betätigung des chirurgischen Instruments 1 wird der Griff 10 zum Handgriff 24 bewegt und schließt die Schere. Zum Öffnen wird der Griff 10 vom Handgriff 24 weg bewegt und öffnet die Schere. Anders als in Figuren 1 und 2 weist das chirurgische Instrument 1 aus Figur 5 keine Rückstellfeder zum Öffnen auf, sondern es weisen der Griff 10 und der Handgriff 24 Fingerringe 39 auf, die mit einem Daumen und einem Zeigefinger durchgegriffen werden, so das sich zum Öffnen der Schere der Griff 10 vom Handgriff 24 weg bewegen lässt. Eine feststehende Klinge 40 der Schere ist starr am Schaft 36 angeordnet. Anstatt einer Schere kann das chirurgische Instrument beispielsweise auch eine Zange oder einen Greifer mit einer schwenkbaren und einer feststehenden Backe anstelle der Klingen aufweisen (nicht dargestellt).

Wie in Figuren 1 und 2 ist an einer Flachseite des Übertragungsglieds 13 der Bedieneinheit 8 des chirurgischen Instruments 1 aus Figur 5 ein Schwenkhebel 19 schwenkbar gelagert, der von einer Blattfeder als Riegelfeder 20 in einer Ausgangsstellung senkrecht zum Übertragungsglied 13 gehalten wird (Figur 6). Der Schwenkhebel 19 ist auf einer Seite des Übertragungsglied 13 in einer Aussparung 35 des Übertragungsglieds 13 angeordnet. Wie in Figuren 1 und 2 weist der Schwenkhebel 19 aus Figuren 5 und 6 einen Rasthaken 21 als Verriegelungselement 23 auf, das mit einem Riegel 22 zusammenwirkt, der seitlich von einem Schwenkhebel 19 absteht, der schwenkbar in einer Aussparung des Handgriffs 24 angeordnet ist, die zur Aussparung 35 im Übertragungsglied 13 offen ist. Auch dieser Schwenkhebel 19 wird von einer Blattfeder als Riegelfeder 20 in seiner Stellung gehalten.

Die Funktion der beiden Schwenkhebel 19 mit dem Rasthaken 21 und dem Riegel 22 als Verriegelungselemente 23 oder als Verriegelunselement 23 und als Gegenstück des Verriegelungselements 23 ist wie anhand der Figuren 1 bis 4 erläutert: wird zu einer Betätigung des chirurgischen Instruments 1 der Griff 10 zum Handgriff 24 geschwenkt bzw. allgemein bewegt, gelangt der Rasthaken 21 in Hintergriff am Riegel 22 und hält das chirurgische Instrument 1 in der betätigten Stellung. Figuren 5 und 6 zeigen die betätigte und verriegelte Stellung. Zum Lösen wird der Griff 10 ein kurzes Stück weiter in Richtung des Handgriffs 24 bewegt, wodurch der Rasthaken 21 außer Eingriff vom Riegel 22 gelangt und eine Verriegelungseinrichtung 18 des chirurgischen Instruments 1 gelöst ist. Durch Entfernen der Fingerringe 39 voneinander wird der Griff 10 vom Handgriff 24 weg bewegt und das chirurgische Instrument 1 geöffnet bzw. in eine nicht betätigte Stellung verbracht. Die beiden Schwenkhebel 19 mit dem Rasthaken 21 und dem Riegel 22 bilden die lösbare mechanische Verriegelungseinrichtung 18 der Bedieneinheit 8 bzw. des chirurgischen Instruments 1.

Die seitliche Abstützung des Übertragungsglieds 13 im Schlitz 12 des Handgriffs 24 verhindert eine Seitwärtsbewegung des Übertragungsglieds 13 und stellt ein zuverlässiges in Eingriff Treten bzw. in Hintergriff Gelangen des Rasthakens 21 am Riegel 22 sicher, die die Verriegelungselemente 23 der Verriegelungseinrichtung 18 bilden. Das heißt die seitliche Abstützung des Übertragungsglieds 13 im Schlitz 12 des Handgriffs 23 stellt eine zuverlässige Verriegelung des chirurgischen Instruments 1 in einer betätigten Stellung beim Bewegen des Griffs 10 zum Handgriff 24 sicher. Das Zwischenglied 16 gleicht eine Seitwärtsbewegung des Griffs 10 bei einer etwaigen seitlichen Kraftbeaufschlagung aus, so dass sich das Übertragungsglied 13 nicht zur Seite bewegt.

## Patentansprüche

1. Chirurgisches instrument (1) mit einer lösbaren mechanischen Verriegelungseinrichtung (18) zur Verwendung in der minimal-invasiven Chirurgie, mit einer Bedieneinheit (8) zu einer Betätigung des Instruments (1) von Hand, die einen Grundkörper (2), einen in Bezug auf den Grundkörper (2) beweglichen Griff (10), ein in Bezug auf den Grundkörper(2) bewegliches Übertragungselement (7) zur Übertragung einer Bewegung des Griffs (10) auf ein Arbeitselement des chirurgischen Instruments (1), und ein Übertragungsglied (13), das mit dem Griff (10) und mit dem Übertragungselement (7) gekoppelt ist und eine Bewegung des Griffs (10) auf das Übertragungselement (7) überträgt, aufweist, wobei ein Verriegelungselement (23) der Verriegelungseinrichtung (18) an dem Übertragungsglied (13) angeordnet ist **dadurch gekennzeichnet, dass** das Verriegelungselement (21,23) an einem Schwenkhebel angeordnet ist, der schwenkbar an dem Übertragungsglied (13) gelagert ist.

2. Chirurgisches Instrument nach Anspruch 1, **dadurch gekennzeichnet, dass** die Bedieneineinheit (8) zwei bewegliche Griffe (10), die einander entgegengesetzt und in Bezug auf den Grundkörper (2) beweglich sind, und zwei Übertragungsglieder (13), von denen jedes mit einem Griff (10) und die beide mit dem Übertragungselement (7) gekoppelt sind und entgegengesetzte Bewegungen der Griffe (10) auf das Übertragungselement (7) übertragen, aufweist.

3. Chirurgisches Instrument nach Anspruch 1 oder 2, **dadurcht gekennzeichnet, dass** der Griff (10) schwenkbar mit dem Grundkörper (2) verbunden und/oder das Übertragungsglied (13) schwenkbar am Griff (10) und/oder am Übertragungselement (7) angelenkt ist.

4. Chirurgisches Instrument nach einem der Ansprüche 1 bis 3, **dadurch gekennzeichnet, dass** das Übertragungselement (7) verschieblich am Grundkörper (2) geführt ist.

5. Chirurgisches Instrument nach Anspruch 2, **dadurch gekennzeichnet, dass** das Verriegelungselement (21, 23) an einem der beiden Übertragungsglieder (13) und ein Gegenstück (22, 23) des Verriegelungselements (21) an einem anderen der beiden Übertragungsglieder (13) angeordnet ist.

6. Chirurgisches Instrument nach einem der vorhergehenden Ansprüche, **dadurch gekennzeichnet, dass** die Bedieneinheit (8) ein Zwischenglied (16) aufweist, das mit dem Griff (10) und mit dem Übertragungsglied (13) gekoppelt ist und eine Bewegung des Griffs (10) auf das Übertragungsglied (13) überträgt.

7. Chirurgisches Instrument nach einem der vorhergehenden Ansprüche, **dadurch gekennzeichnet, dass** der Grundkörper (2) einen Schlitz (12) aufweist, in dem das/die Übertragungsglieder (13) zumindest teilweise aufgenommen sind und der so breit wie das Übertragungsglied (13) dick ist oder die beiden Übertragungsglieder (13) zusammen dick sind.

## Claims

1. Surgical instrument (1) having a detachable mechanical locking device (18) for use in minimally invasive surgery, comprising a control unit (8) for actuating the instrument (1) by hand, which unit has a main body (2), a handle (10) that can be moved relative to the main body (2), a transmission element (7), which can be moved relative to the main body (2), for transmitting a movement of the handle (10) to a working element of the surgical instrument (1), and a transmission member (13) that is coupled to the handle (10) and the transmission element (7) and transmits a movement of the handle (10) to the transmission element (7), a locking element (23) of the locking device (18) being arranged on the transmission member (13), **characterised in that** the locking element (21, 23) is arranged on a pivot lever that is pivotably mounted on the transmission member (13).

2. Surgical instrument according to claim 1, **characterised in that** the control unit (8) comprises two movable handles (10), which can be moved in opposite directions and relative to the main body (2), and two transmission members (13), each of which is coupled to a handle (10) and both of which are coupled to the transmission element (7) and transmit opposing movements of the handles (10) to the transmission element (7).

3. Surgical instrument according to either claim 1 or claim 2, **characterised in that** the handle (10) is pivotably connected to the main body (2) and/or the transmission member (13) is pivotably linked to the handle (10) and/or the transmission element (7).

4. Surgical instrument according to any of claims 1 to 3, **characterised in that** the transmission element (7) is movably guided on the main body (2).

5. Surgical instrument according to claim 2, **characterised in that** the locking element (21, 23) is arranged on one of the two transmission members (13) and a mating part (22, 23) of the locking element (21) is arranged on the other of the two transmission members (13).

6. Surgical instrument according to any of the preceding claims, **characterised in that** the control unit (8) comprises an intermediate member (16) that is coupled to the handle (10) and to the transmission member (13) and transmits a movement of the handle (10) to the transmission member (13).

7. Surgical instrument according to any of the preceding claims, **characterised in that** the main body (2) comprises a slot (12) in which the transmission member(s) (13) is/are received at least in part and which is as wide as the transmission member (13) is thick or as the two transmission members (13) together are thick.

## Revendications

1. Instrument chirurgical (1) équipé d'un dispositif (18) de verrouillage mécanique libérable, pour l'utilisation en chirurgie mini-invasive, comportant une unité de manoeuvre (8) dévolue à un actionnement manuel dudit instrument (1) et comprenant un corps de base (2), une poignée (10) mobile par rapport au corps de base (2), un élément de transmission (7) mobile par rapport audit corps de base (2) et destiné à répercuter un mouvement de la poignée (10) sur un élément de travail dudit instrument chirurgical (1), et un organe de transmission (13) qui, couplé à la poignée (10) et à l'élément de transmission (7), répercute un mouvement de ladite poignée (10) sur ledit élément de transmission (7), un élément de verrouillage (23) dudit dispositif de verrouillage (18) étant implanté sur ledit organe de transmission (13), **caractérisé par le fait que** l'élément de verrouillage (21, 23) est situé sur un levier pivotant monté à pivotement sur l'organe de transmission (13).

2. Instrument chirurgical selon la revendication 1, **caractérisé par le fait que** l'unité de manoeuvre (8) compte deux poignées mobiles (10), aptes à se mouvoir en opposition l'une à l'autre et par rapport au corps de base (2), et deux organes de transmission (13) dont chacun est couplé à une poignée (10), qui sont couplés l'un et l'autre à l'élément de transmission (7) et répercutent, sur ledit élément de transmission (7), des mouvements opposés opérés par lesdites poignées (10).

3. Instrument chirurgical selon la revendication 1 ou 2, **caractérisé par le fait que** la poignée (10) est reliée de manière pivotante au corps de base (2) et/ou l'organe de transmission (13) est articulé, de manière pivotante, sur ladite poignée (10) et/ou sur l'élément de transmission (7).

4. Instrument chirurgical selon l'une des revendications 1 à 3, **caractérisé par le fait que** l'élément de transmission (7) est guidé sur le corps de base (2) avec faculté de coulissement.

5. Instrument chirurgical selon la revendication 2, **caractérisé par le fait que** l'élément de verrouillage (21, 23) est situé sur l'un des deux organes de transmission (13), une pièce (22, 23) complémentaire dudit élément de verrouillage (21) étant située sur un autre, parmi les deux organes de transmission (13).

6. Instrument chirurgical selon l'une des revendications précédentes, **caractérisé par le fait que** l'unité de manoeuvre (8) est dotée d'un organe intermédiaire (16) qui est relié à la poignée (10) et à l'organe de transmission (13), et répercute un mouvement de ladite poignée (10) sur ledit organe de transmission (13).

7. Instrument chirurgical selon l'une des revendications précédentes, **caractérisé par le fait que** le corps de base (2) est pourvu d'une fente (12) dans laquelle l'organe/les organes de transmission (13) est (sont) au moins partiellement reçu(s), et dont la largeur équivaut à l'épaisseur de l'organe de transmission (13), ou aux épaisseurs conjuguées des deux organes de transmission (13).
